# EUROPEAN PATENT APPLICATION

(11) **EP 4 620 568 A1**
(43) Date of publication of application: **24.09.2025**
(21) Application number: 22965436.3
(22) Date of filing: 15.11.2022
(51) Int. Cl.: B01J 27/232, C07C 9/06, C07C 2/84, C07C 11/04, C07C 9/08

(54) **LANTHANUM OXYCARBONATE CATALYST, PREPARATION METHOD THEREFOR AND USE THEREOF**

(71) Applicant: China Petroleum & Chemical Corporation, Beijing 100728 (CN); Sinopec (Beijing) Research Institute of Chemical Industry Co., Ltd., Beijing 100013 (CN)
(72) Inventor: WU, Jiehua, Beijing 100013 (CN); XUE, Wei, Beijing 100013 (CN); WANG, Huanru, Beijing 100013 (CN); LIU, Dongbing, Beijing 100013 (CN); WANG, Xue, Beijing 100013 (CN); SHAO, Yun, Beijing 100013 (CN)
(74) Representative: Hoffmann Eitle
(86) International application number: PCT/CN2022/131849
(87) International publication number: WO 2024/103244

(57) **Abstract**

The present invention relates to the technical field of lanthanum oxycarbonate, and discloses a lanthanum oxycarbonate catalyst, a preparation method and an application thereof. The lanthanum oxycarbonate catalyst comprises hexagonal phase lanthanum oxycarbonate, monoclinic phase lanthanum oxycarbonate containing a doping element and tetragonal phase lanthanum oxycarbonate containing a doping element. When the catalyst of the present invention is used for methane oxidative coupling reaction, it has a high C2 hydrocarbon yield at low temperature.

## Description

### Technical Field

The present invention relates to the technical field of lanthanum oxycarbonate, and in particular to a lanthanum oxycarbonate catalyst and a preparation method and application thereof.

### Background Art

Ethylene is an important basic organic chemical raw material. For a long time, its production has been dependent on the steam cracking route of petroleum fractions, and the resulting environmental pollution and other problems are becoming increasingly serious. In recent years, crude oil prices have continued to rise, leading to an increase in the price of ethylene cracking raw materials. At the same time, the shortage of ethylene cracking raw materials is also very prominent. Faced with this situation, countries around the world are adjusting their energy utilization structure and constantly looking for new ethylene production routes. Oxidative coupling of methane (OCM), which is a one-step method for producing ethylene and ethane (collectively referred to as C2 hydrocarbons) from methane, has been widely studied as a method for directly producing ethylene and ethane. This method is environmentally friendly. Since it was proposed in 1982, researchers have conducted a lot of researches on catalysts, catalytic processes and reactors. Various catalyst systems about the OCM reaction have been developed from the perspective of revealing active centers, improving the yield of product C2 hydrocarbons and improving the stability of catalysts. In the past four decades, a large number of catalysts have been developed, such as Mn₂O₃ -Na₂WO₄ /SiO₂, perovskite-type systems, MgO-based catalysts, supported La materials and nano-scale La-based catalysts. However, there are still some problems that limit the commercialization of the OCM process: (1) high reaction temperature (700-900 °C) is required to achieve high production of C2 hydrocarbons; (2) the concentration of C2 hydrocarbons in the product is low, which makes the separation cost of the product high; and (3) when the methane conversion rate is high, the selectivity of the corresponding C2 hydrocarbons is low, making it very difficult to achieve both high methane conversion and C2 hydrocarbons selectivity at the same time.

The prior art does not mention how to prepare a catalyst containing hexagonal phase lanthanum oxycarbonate, monoclinic phase lanthanum oxycarbonate and tetragonal phase lanthanum oxycarbonate at the same time by a one-step method, nor does it discuss the effect of lanthanum oxycarbonate having these three crystal phase structures on the catalytic performance.

### Summary of the invention

The purpose of the present invention is to overcome the above-mentioned problems existing in the prior art and to provide a lanthanum oxycarbonate catalyst and a preparation method and application thereof.

In order to achieve the above-mentioned object, in a first aspect, the present invention provides a lanthanum oxycarbonate catalyst, which includes the following three crystal phases coexisting in a crystalline state: hexagonal phase lanthanum oxycarbonate, monoclinic phase lanthanum oxycarbonate containing a doping element, and tetragonal phase lanthanum oxycarbonate containing a doping element. Preferably, the ratio of the molar amount of the hexagonal phase lanthanum oxycarbonate to the total molar amount of the tetragonal phase lanthanum oxycarbonate and the monoclinic phase lanthanum oxycarbonate is 1:0.01-1. Also preferably, the molar ratio of the tetragonal phase lanthanum oxycarbonate and the monoclinic phase lanthanum oxycarbonate is 1:0.1-10, preferably 1:0.2-5, or 1:0.25-3.

On the other hand, the present invention provides a method for preparing the lanthanum oxycarbonate catalyst, which comprises: adding an alkali solution to a mixed solution containing a lanthanum source and a compound containing a doping element to make the pH value of the mixed system greater than 9, and then sequentially performing reaction, solid-liquid separation, drying and calcination; wherein, relative to each kilogram of the mixed solution, the addition rate of the alkali solution is 0.01 g/min-10 g/min, preferably 0.03 g/min-8 g/min, and more preferably 0.5 g/min-6 g/min, based on the alkali in the alkali solution; and wherein, firstly, the alkali solution is added dropwise to the mixed solution at a first addition rate until the pH value of the solution reaches a set value, which is between 8 and 9, and then the alkali solution is added dropwise to the mixed solution at a second addition rate until the pH value of the system is 10-13, wherein the second addition rate is 0.5-4.5 g/min higher than the first addition rate.

In another aspect, the present invention provides a lanthanum oxycarbonate catalyst prepared by the method described in the present invention.

In another aspect, the present invention also provides a lanthanum oxycarbonate catalyst composition formed by combining the lanthanum oxycarbonate catalyst of the present invention with a binder or a support.

In another aspect, the present invention provides the use of the lanthanum oxycarbonate catalyst of the present invention in the oxidative coupling reaction of methane to produce C2+ hydrocarbons.

In another aspect, the present invention provides a method for preparing C2+ hydrocarbons from methane, comprising: contacting methane with the lanthanum oxycarbonate catalyst of the present invention for reaction in the presence of oxygen and under the conditions of methane oxidative coupling reaction.

The present invention proposes for the first time a catalyst comprising hexagonal phase lanthanum oxycarbonate, monoclinic phase lanthanum oxycarbonate containing a doping element, and tetragonal phase lanthanum oxycarbonate containing a doping element, wherein the catalyst is prepared by controlling the formation conditions of the crystal phase by adding an alkali solution to a mixed solution containing a lanthanum source and a compound containing a doping element. The contents of the monoclinic phase lanthanum oxycarbonate and the tetragonal phase lanthanum oxycarbonate in the catalyst can be regulated by the amount of doping element added and the formation conditions of the crystal phase (for example, controlling the addition rate of the alkali solution and the reaction conditions). When the catalyst of the present invention is used for the methane oxidative coupling reaction, it has a high yield of C2 hydrocarbons at low temperature (450-650 °C).

### Description of Drawings

FIG 1 is a scanning electron microscope (SEM) image of the lanthanum oxycarbonate catalyst prepared in Example 1;
FIG 2 is an X-ray (XRD) spectrum of the lanthanum oxycarbonate catalyst prepared in Example 1;
FIG 3 is an X-ray (XRD) spectrum of the lanthanum oxycarbonate catalyst prepared in Example 4;
FIG 4 is an X-ray (XRD) spectrum of the lanthanum oxycarbonate catalyst prepared in Example 6;
FIG 5 is an X-ray (XRD) spectrum of the lanthanum oxycarbonate catalyst prepared in Example 7;
FIG 6 is an X-ray (XRD) spectrum of the lanthanum oxycarbonate catalyst prepared in Comparative Example 1;

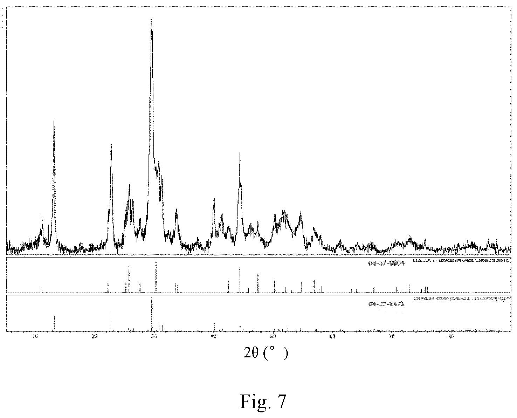

FIG 7 is an X-ray (XRD) spectrum of the lanthanum oxycarbonate catalyst prepared in Comparative Example 3.

### Specific Mode for Carrying out the Invention

The endpoints and any values of the ranges disclosed herein are not limited to the precise ranges or values, and these ranges or values should be understood to include values close to these ranges or values. For numerical ranges, the endpoint values of each range, the endpoint values of each range and the individual point values, and the individual point values can be combined with each other to obtain one or more new numerical ranges, which should be considered as specifically disclosed in the present application.

In the present application, the "coexisting in a crystalline state" means that unlike simple mechanical mixing, the three crystal phases of hexagonal phase, monoclinic phase and tetragonal phase are not only uniformly mixed at the macroscopic scale, but also uniformly mixed at the microscopic scale. Specifically, the three crystal phases described in the present invention can be measured by XRD test; accordingly, the "coexisting in a crystalline state" described in the present invention is that when the XRD test of the sample of the product of the present invention is carried out, each unit weight of the test sample required for the test measures the simultaneous existence of the three crystal phases. For the purpose of the present invention, for example, the "unit weight" of the test sample is not more than 1g, not more than 0.5g, or preferably not more than 0.3g. It is also preferred that for the product of the present invention, the unit weight of the test sample subjected to the XRD test is at least 3 parts, or at least 5 parts, or at least 10 parts.

For example, in the present invention, a batch of the products produced by the method of the present invention can be divided into several test samples of unit weight required for measurement, evenly or unevenly in weight; or several batches of the products produced by the method of the present invention can be divided into several test samples of unit weight required for measurement, evenly or unevenly in weight; or several batches of the products produced by the method of the present invention can also be mixed with each other and then divided into several test samples of unit weight required for measurement, evenly or unevenly in weight.

In the present application, the "C2" hydrocarbon refers to hydrocarbons with 2 carbon atoms, especially olefins and alkanes, such as ethylene and ethane. The remaining hydrocarbons represented by carbon number each have similar meanings.

In a first aspect, the present invention provides a lanthanum oxycarbonate catalyst, which includes the following three crystal phases coexisting in a crystalline state: hexagonal phase lanthanum oxycarbonate, monoclinic phase lanthanum oxycarbonate containing a doping element, and tetragonal phase lanthanum oxycarbonate containing a doping element.

According to the present invention, preferably, the doping element in the monoclinic phase lanthanum oxycarbonate and the tetragonal phase lanthanum oxycarbonate is selected from at least one of the elements of Groups IIA, VIII, IB, IIB and lanthanide elements other than lanthanum, preferably at least one of Mg, Ca, Sr, Ba, Fe, Zn, Ce and Cd, and more preferably at least one of Mg, Ca, Sr and Ba.

According to the present invention, preferably, the molar ratio of lanthanum element to the doping element in the lanthanum oxycarbonate catalyst is 1:0.01-0.1, preferably 1:0.01-0.08, and more preferably 1:0.012-0.06.

According to the present invention, preferably, the ratio of the molar amount of the hexagonal phase lanthanum oxycarbonate to the total molar amount of the tetragonal phase lanthanum oxycarbonate and the monoclinic phase lanthanum oxycarbonate is 1:0.01-1, preferably 1:0.1-1, and more preferably 1:0.25-0.9, wherein, the content of each crystal phase in the catalyst is calculated according to the XRD full spectrum fitting method ( Rietveld method ), specifically using Highscore plus 4.0 software for standard-free quantitative calculation, first determining the background, peak search, and object matching, then inserting the structural information (unit cell parameters) of the hexagonal phase lanthanum oxycarbonate and the monoclinic phase lanthanum oxycarbonate, and automatically and manually refining the global parameters and the parameters of each component, the error parameter (Rwp) is as small as possible, and when Rwp is less than 10, the quantification is completed, and the computer outputs the calculation result (i.e., the ratio of the molar amount of the hexagonal phase lanthanum oxycarbonate to the total molar amount of the tetragonal phase lanthanum oxycarbonate and the monoclinic phase lanthanum oxycarbonate).

For the purpose of the present invention, for the convenience of calculation, as described above, when investigating the molar relationship of the three crystal phases of hexagonal phase, monoclinic phase and tetragonal phase, the total molar amount of the tetragonal phase lanthanum oxycarbonate and the monoclinic phase lanthanum oxycarbonate is investigated, wherein the unit cell parameters of the monoclinic phase lanthanum oxycarbonate are used as representatives of the unit cell parameters of the tetragonal phase lanthanum oxycarbonate. Accordingly, in the XRD full spectrum fitting method calculation process, the crystal structure data of the hexagonal phase lanthanum oxycarbonate and the structure data of the monoclinic phase lanthanum oxycarbonate are assigned as initial values. In the present invention, the standard spectrum of the XRD of the hexagonal phase lanthanum oxycarbonate is PDF:00-37-0804, the standard spectrum of the XRD of the tetragonal phase lanthanum oxycarbonate is PDF:00-23-0320, and the standard spectrum of the XRD of the monoclinic phase lanthanum oxycarbonate is PDF 04-22-8421.

Surprisingly, the XRD spectrum of the catalyst prepared by element doping using the specific method of the present invention clearly shows that lanthanum oxycarbonate of different crystal phases is formed, and in particular, lanthanum oxycarbonate of hexagonal phase, monoclinic phase and tetragonal phase coexisting in a crystalline state.

It has been speculated by those skilled in the art that there is a possibility of interconversion between the tetragonal phase and the monoclinic phase of lanthanum oxycarbonate, however, the inventors have not seen any literature or other public reports to confirm this speculation, and have not seen any research providing relevant data and/or other experimental evidence. Accordingly, the inventors have not seen any reports providing lanthanum oxycarbonate described in the present invention in which the three crystal phases coexist in a crystalline state, especially, for example, quantitative analysis of the three crystal phases to confirm that there are indeed three crystal phases coexisting, and to analyze the amount of one or more of them.

Accordingly, as measured by the XRD spectrum, the ratio R1 of the molar amount of the hexagonal phase lanthanum oxycarbonate to the total molar amount of the tetragonal phase lanthanum oxycarbonate and the monoclinic phase lanthanum oxycarbonate is 1:0.01-1.

Preferably, the molar ratio R2 of the tetragonal phase lanthanum oxycarbonate and the monoclinic phase lanthanum oxycarbonate is 1:0.1-10, preferably 1:0.2-5, or 1:0.25-3. According to one embodiment of the present invention, the ratio R2 is based on the XRD spectrum, and the relative contents of the two structures are determined by peak separation by a spectrum fitting method; the method for peak separation is known in the art. Preferably, the ratio R2 is based on the XRD spectrum used to measure the ratio R1. After calculation and statistics, the calculation error of the fitting calculation of the ratio R2 is less than or equal to 25%.

According to the present invention, the lanthanum oxycarbonate catalyst can be particles with a nanostructure, especially when it is prepared by the method of the present invention. In the art, the morphology of a solid can be described in three dimensions. In this regard, regardless of the specific morphology of the catalyst particles, when the smallest dimension of the three-dimensional scale is at the nanometer level, for example, not exceeding 1000nm, preferably not exceeding 500nm, it belongs to the "nano" structure described in the present invention. For example, it is widely known in the art that for catalyst particles of different morphologies, dimensions such as aspect ratio and thickness are often used to provide descriptions; accordingly, in the present application, as long as the diameter or thickness of the particle is at the nanometer level, it has the "nano" structure described in the present invention.

In the present invention, the "diameter" and "aspect ratio" of the lanthanum oxycarbonate catalyst particles are both average values. The specific test method for the "average diameter" is to use a transmission electron microscope scale, select 5-10 samples in the window, measure the diameter of each sample, and then calculate the average value. The specific test method for the "average aspect ratio" is to use a transmission electron microscope scale, select 5-10 samples in the window, measure the diameter and length of each sample respectively, and then calculate the aspect ratio of each sample, and then calculate the average value.

According to the present invention, preferably, the lanthanum oxycarbonate catalyst particles have a rod-like nanostructure. It is understood by those skilled in the art that, for catalyst particles, the present invention refers to "rod-like" in accordance with the conventional understanding of the art, and does not have to be a rod-like in a strict geometric sense. For example, the particle forms commonly seen in the art and described as oblong, elliptical, needle-like, etc., also belong to the "rod-like" structure described in the present invention, as long as they meet the diameter and /or aspect ratio requirements described in the present invention. Specifically, in one embodiment, the diameter of the rod-like nanostructured lanthanum oxycarbonate catalyst is 10nm-30nm (for example, it can be 10nm, 12nm, 14nm, 15nm, 18nm, 20nm, 22nm, 24nm, 26nm, 28nm, 30nm, and ranges composed of any two of the above points). In one embodiment, the aspect ratio of the rod-like nanostructured lanthanum oxycarbonate catalyst is 5-30:1 (e.g., 5:1, 7:1, 9:1, 11:1, 13:1, 15:1, 17:1, 19:1, 20:1, 22:1, 24:1, 26:1, 28:1, 30:1, and ranges composed of any two of the above points), and more preferably 10-26:1.

According to the present invention, preferably, the specific surface area of the rod-like nanostructured lanthanum oxycarbonate catalyst is 40 m²/g- 100 m²/g (for example, it can be 40 m²/g, 50 m²/g, 60 m²/g, 65 m²/g, 70 m²/g, 75 m²/g, 80 m²/g, 85 m²/g, 90 m²/g, 100 m²/g, and ranges composed of any two of the above points). In one embodiment, the pore volume of the rod-like nanostructured lanthanum oxycarbonate catalyst is 0.2 cm³/g - 0.7 cm³/g (for example, it can be 0.2 cm³/g, 0.3 cm³/g, 0.35 cm³/g, 0.4 cm³/g, 0.45 cm³/g, 0.5 cm³/g, 0.55 cm³/g, 0.6 cm³/g, 0.65 cm³/g, 0.7 cm³/g, and ranges composed of any two of the above points). In one embodiment, the average pore size of the rod-like nanostructured lanthanum oxycarbonate catalyst is 5 nm-300 nm (for example, it can be 5 nm, 8 nm, 10 nm, 10.5 nm, 11 nm, 11.5 nm, 12 nm, 12.5 nm, 13 nm, 15 nm, 20 nm, 25 nm, 30 nm, 50 nm, 80 nm, 100 nm, 150 nm, 160 nm, 170 nm, 180 nm, 190 nm, 200 nm, 210 nm, 220 nm, 230 nm, 240 nm, 250 nm, 260 nm, 270 nm, 280 nm, 290 nm, 300 nm, and ranges composed of any two of the above points), more preferably 8 nm -290 nm, and more preferably 180 nm -260 nm.

According to the present invention, the lanthanum oxycarbonate catalyst particles may have a flake structure. It is understood by those skilled in the art that, for catalyst particles, the present invention refers to "flake" in accordance with the conventional understanding of the art. For example, a "flake" structure may generally refer to a structural form in which the scale in the "thickness" direction is significantly smaller than the scales in the other two dimensional directions. For the purposes of the present invention, the plane formed by the two dimensions other than the thickness direction of the flake structure particles is referred to as the "cross-section" of the particles. For the purposes of the present invention, the flake structure particles may have cross-sections of various shapes, such as circular, elliptical, parallelogram, or shapes close to these shapes. Preferably, for example, the flake structure particles of the present invention may have a "nearly parallelogram" cross-section.

According to the present invention, the term "nearly parallelogram" includes a parallelogram and a quadrilateral close to a parallelogram, that is, the sum of two adjacent internal angles of the nearly parallelogram is equal to or close to 180° .

Preferably, two adjacent interior angles of the nearly parallelogram are recorded as ∠A and ∠B*,* wherein ∠A is set to be an acute angle or a right angle, ∠B is set to be an obtuse angle or a right angle, 60° < ∠ A<90° , 170 ° <(∠A+∠B)<195° . For example, ∠A can be 65° , 68° , 70° , 72° , 75° , 78° , 80° , 82° , 85° , 88° . ∠A+∠B can be 172° , 175° , 178° , 180° , 182° , 185° , 188° , 190° , 193° .

According to the present invention, a rectangular or square cross section also belongs to the "nearly parallelogram" cross section.

The angle can be measured by using an angle measuring scale provided by a scanning electron microscope.

According to the present invention, the side length of the nearly parallelogram may vary within a wide range, preferably 1 µm-5 µm, for example, 1 µm, 2 µm, 3 µm, 4 µm, 5 µm, preferably 1.5 µm-3.5 µm.

The side length can be measured by a scale provided by a scanning electron microscope.

According to the present invention, preferably, the thickness of the nearly parallelogram lanthanum oxycarbonate can vary within a wide range, preferably 100 nm - 500 nm, for example, 100 nm, 200 nm, 300 nm, 400 nm, 500 nm, preferably 200 nm - 400 nm.

The thickness can be measured by a scale provided by a scanning electron microscope.

According to the present invention, the lanthanum oxycarbonate catalyst particles may have a spherical structure. It is understood by those skilled in the art that, for catalyst particles, the present invention refers to "spherical" in accordance with conventional knowledge in the art. For example, a "spherical" structure generally refers to particles having a sphericity greater than 0.5, greater than 0.6, greater than 0.7, greater than 0.8, or greater than 0.9.

According to the present invention, preferably, the diameter of the spherical lanthanum oxycarbonate can vary within a wide range, preferably 100 nm - 800 nm, for example 200 nm - 600 nm.

For the purposes of the present invention, it is preferred that the lanthanum oxycarbonate catalyst of the present invention has a rod-like nanostructure.

In another aspect, the present invention provides a method for preparing the lanthanum oxycarbonate catalyst particles of the present invention, comprising: adding an alkali solution to a mixed solution containing a lanthanum source and a compound containing a doping element, so that the pH value of the mixed system is greater than 9 (the pH value at this time refers to the pH value of the mixed system when the addition of the alkali solution is stopped), and then sequentially reacting, solid-liquid separation, drying and calcining. In the present invention, the reaction described herein specifically refers to the reaction of a lanthanum nitrate solution with an alkali solution to generate solid lanthanum hydroxide.

The inventors of the present invention have found in their research that by adding alkali solution to a mixed solution containing a lanthanum source and a compound containing a doping element, and then sequentially reacting, solid-liquid separation, drying and calcining, a relatively stable hexagonal phase lanthanum oxycarbonate can be partially converted into a relatively less stable tetragonal phase and monoclinic phase lanthanum oxycarbonate, and the three phases exist in a relatively stable mixed form. The presence of this mixed crystal phase can increase the low-temperature activation performance of the catalyst.

According to the present invention, the lanthanum source can be any substance that can provide lanthanum element. Preferably, the lanthanum source is a water-soluble salt of lanthanum, more preferably at least one of lanthanum nitrate, lanthanum chloride and lanthanum acetate, and further preferably lanthanum nitrate.

According to the present invention, the type of the compound containing the doping element can be selected in a wide range, as long as the required doping element can be provided. Preferably, the compound containing the doping element is a compound containing at least one element selected from Groups IIA, VIII, IB, IIB and lanthanide elements other than lanthanum, more preferably a compound containing at least one element selected from Mg, Ca, Sr, Ba, Fe, Zn and Cd, and further preferably a compound containing at least one element selected from Mg, Ca, Sr and Ba. The compound containing the doping element can be at least one of nitrates (e.g., magnesium nitrate, calcium nitrate, strontium nitrate, barium nitrate, iron nitrate, zinc nitrate, cadmium nitrate), chlorides (e.g., magnesium chloride, calcium chloride, strontium chloride, barium chloride, iron chloride, zinc chloride, cadmium chloride) and acetates (e.g., magnesium acetate, calcium acetate, strontium acetate, barium acetate, iron acetate, zinc acetate, cadmium acetate) of the doping element, preferably nitrates.

According to the present invention, the amounts of the lanthanum source and the compound containing the doping element can be selected in a wide range, but in order to increase the yield of C2 hydrocarbons and reduce the temperature of the methane oxidative coupling reaction, and at the same time to reduce the preparation cost, preferably, the amounts of the lanthanum source and the compound containing the doping element are such that the molar ratio of lanthanum element to doping element in the lanthanum oxycarbonate catalyst is 1:0.01-0.1, more preferably 1:0.01-0.08, and further preferably 1:0.012-0.06.

According to the present invention, preferably, the amounts of the lanthanum source and the compound containing the doping element are such that the ratio of the molar amount of the hexagonal phase lanthanum oxycarbonate to the total molar amount of the tetragonal phase lanthanum oxycarbonate and the monoclinic phase lanthanum oxycarbonate in the lanthanum oxycarbonate catalyst is 1:0.01-1, preferably 1:0.1-1, and more preferably 1:0.25-0.9.

According to the present invention, the main purpose of the alkali solution is to provide an alkaline environment for the system, and thus it can be any liquid capable of providing alkaline conditions, for example, aqueous ammonia, hydroxide solution, carbonate solution, bicarbonate solution. Without being limited by any known theory, it is believed that the selection of a specific alkali for the alkali solution helps to control the crystal morphology of the resulting product. Accordingly, it is preferred that the alkali in the alkali solution is a compound of Group IA metal; more preferably, the alkali is sodium hydroxide and/or potassium hydroxide; without being limited by any known theory, it is believed that such alkali is particularly conducive to the formation of rod-shaped nanostructured lanthanum oxycarbonate particles.

According to the present invention, preferably, the amount of the alkali solution makes the pH value of the mixed system >9, more preferably 10-13. Without being limited by any known theory, it is believed that the pH value of the mixed system is particularly conducive to the formation of rod-like nanostructured lanthanum oxycarbonate particles.

According to the present invention, preferably, the concentration of the alkali solution is 2wt%-25wt%, preferably 3wt%-20wt%, and more preferably 5wt%-18wt%. Without being limited to any known theory, it is believed that the concentration of the alkali solution in the mixed system is particularly conducive to the formation of rod-shaped nanostructured lanthanum oxycarbonate particles.

According to the present invention, preferably, the method further comprises:
S1. under a first stirring condition, mixing the lanthanum source and the compound containing the doping element with water to obtain a mixed solution; preferably, the first stirring condition includes: a temperature of 10-50 °C, a rotation speed of 300-1000rpm, and a time of 10-40min;
S2. under a second stirring condition, adding the alkali solution to the mixed solution to obtain a mixed system; preferably, the second stirring condition includes: a temperature of 20-60 °C and a rotation speed of 500-1500 rpm.

According to the present invention, a specific alkali solution addition rate is adopted. Preferably, relative to per kilogram of the mixed solution, the alkali solution addition rate is 0.01 g/min- 10 g/min, preferably 0.03 g/min-8 g/min, and more preferably 0.5 g/min-6 g/min, calculated as the compound of the Group IA metal.

According to the specific alkali solution addition rate of the present invention, in order to further increase the content of the doping element in the lanthanum oxycarbonate catalyst, preferably, the alkali solution addition rate during adding the alkali solution to the mixed solution containing the lanthanum source and the compound containing the doping element is controlled. Specifically, in step S2, under the second stirring condition, the alkali solution is added dropwise to the mixed solution containing the lanthanum source and the compound containing the doping element at a first addition rate until the pH of the solution reaches a set value, which is between 8 and 9, and then the alkali solution is added dropwise to the mixed solution containing the lanthanum source and the compound containing the doping element at a second addition rate until the pH of the system is 10-13, and the addition of the alkali solution is stopped.

More preferably, in step S2, the second addition rate is 0.5-4.5 g/min (for example, 0.5 g/min, 1 g/min, 1.5 g/min, 2 g/min, 2.5 g/min, 3 g/min, 3.5 g/min, 4 g/min, 5.5 g/min, 5 g/min, and ranges consisting of any two of the above values) higher than the first addition rate.

More preferably, in step S2, under the second stirring condition, the alkali solution is added dropwise to the mixed solution comprising the lanthanum source and the compound containing the doping element at a first addition rate of 0.01-1 g/min (for example, 0.01 g/min, 0.1 g/min, 0.2 g/min, 0.3 g/min, 0.4 g/min, 0.5 g/min, 0.6 g/min, 0.7 g/min, 0.8 g/min, 0.9 g/min, 1 g/min, and ranges composed of any two of the above points) per kilogram of the mixed solution comprising the lanthanum source and the compound containing the doping element, in terms of the compound of the Group IA metal, until the pH of the solution reaches a set value, which is between 8 and 9, and then the alkali solution is added dropwise to the mixed solution comprising the lanthanum source and the compound containing the doping element at a second addition rate of 0.5-5 g/min (for example, 0.5 g/min, 0.6 g/min, 0.7 g/min, 0.8 g/min, 0.9 g/min, 1 g/min, 1.5 g/min, 2 g/min, 2.5 g/min, 3 g/min, 3.5 g/min, 4 g/min, 4.5 g/min, 5 g/min, and ranges consisting of any two of the above points) per kilogram of the mixed solution comprising the lanthanum source and the compound containing the doping element, in terms of the compound of the Group IA metal, and the addition of the alkali solution is stopped when the pH of the system is 10-13.

Without being limited to any known theory, it is believed that the specific alkali solution addition rate of the present invention is particularly conducive to the formation of multiple crystal forms required by the present invention. For example, the specific alkali solution addition rate of the present invention is believed to be conducive to controlling the supersaturation of the solution.

According to the present invention, preferably, the reaction temperature is 80-200 °C (for example, it can be 80 °C, 85°C, 90 °C, 95 °C, 100 °C, 120 °C, 130 °C, 140 °C, 150 °C, 160 °C, 170 °C, 180 °C, 190 °C, 200 °C, and ranges composed of any two of the above points), and the time is 10h-50h ( for example, it can be 10h, 15h, 20h, 25h, 30h, 35h, 40h, 45h, 50h, and ranges composed of any two of the above points).

According to the present invention, the reaction can be carried out by an aging manner or a hydrothermal manner.

According to the present invention, preferably, the aging is carried out under stirring, and the stirring speed is 500-1500 rpm. The present invention has no particular limitation on the aging device, as long as the aging temperature and stirring conditions can be met, but based on the safety of the experiment and the comprehensive catalyst performance, the aging is carried out under condensation reflux. More preferably, the aging conditions include a temperature of 80-100 °C and a time of 10 h-50 h. Aging is carried out under normal pressure (0.1MPa).

Preferably, the hydrothermal conditions include a temperature of 100-200 °C. and a time of 10 h-50 h. The hydrothermal pressure is the pressure generated by the hydrothermal reaction itself.

According to the present invention, preferably, the method further comprises: before drying, washing the aged product, wherein the washing solvent is water and/or a C1-C4 monohydric alcohol. According to a preferred embodiment of the present invention, the aged product is first washed with water (distilled water) until neutral, and then washed with ethanol for 1-2 times.

According to the present invention, the drying conditions can be changed within a wide range. Preferably, the drying temperature is 60 °C-100 °C, for example, it can be 60 °C, 70 °C, 80 °C, 90 °C, 100 °C, and the drying time is 12h -24h, for example, it can be 12h, 14h, 16h, 18h, 20h, 22h, 24h.

According to the present invention, the calcination conditions can be changed within a wide range. Preferably, the calcination temperature is 450 °C -550 °C, for example, it can be 450 °C, 460 °C, 470 °C, 480 °C, 490 °C, 500 °C, 510 °C, 520 °C, 530 °C, 550 °C, and the calcination time is 2h-8h, for example, it can be 2h, 3h, 4h, 5h, 6h, 7h, 8h.

According to the present invention, the calcination atmosphere is not particularly limited and may be an air atmosphere or a carbon dioxide atmosphere.

In another aspect, the present invention provides a lanthanum oxycarbonate catalyst prepared by the method described in the present invention.

In another aspect, the present invention provides the use of the lanthanum oxycarbonate catalyst of the present invention in the oxidative coupling reaction of methane to produce C2+ hydrocarbons.

The lanthanum oxycarbonate catalyst prepared by the method of the present invention is preferably a nanostructured particle, which can be used directly, such as in the application of methane oxidative coupling reaction to produce C2+ hydrocarbons. However, for reasons such as efficiency of use, engineering requirements, such as in the application of methane oxidative coupling reaction to produce C2+ hydrocarbons, the lanthanum oxycarbonate catalyst can be further processed and formed, especially, for example, when used in a fixed bed reactor. For example, the lanthanum oxycarbonate particles of the present invention can be tableted to have a size of, for example, 40-60 mesh.

The lanthanum oxycarbonate particles of the present invention may be used in combination with a binder, which may be those conventionally used in the art, such as alumina, soluble starch, silica sol, polyethylene glycol, and the like.

The lanthanum oxycarbonate particles of the present invention may be supported on a support. The support may be those conventionally used in the art, such as alumina, silica, molecular sieves (mesoporous molecular sieves, microporous molecular sieves), diatomaceous earth, ceramics, and the like.

In another aspect, the present invention provides a method for preparing C2+ hydrocarbons from methane, comprising: contacting methane with the lanthanum oxycarbonate catalyst of the present invention for reaction in the presence of oxygen and under the conditions of methane oxidative coupling reaction.

According to the present invention, the conditions of the methane oxidative coupling reaction are not particularly limited and can be selected conventionally in the art. The conditions of the methane oxidative coupling reaction can include: a molar ratio of methane to oxygen of 2-9:1, a contact and reaction temperature of 450 °C -650 °C (for example, 450 °C, 460 °C, 470 °C, 480 °C, 490 °C, 500 °C, 520 °C, 530 °C, 550 °C, 570 °C, 580 °C, 600 °C, 650 °C), and a space velocity of methane of 5000 mL/(g·h)-200000 mL/(g·h).

The present invention will be described in detail below by way of examples. In the following examples,
TEM imaging was performed using a JEOL 2100F FEG TEM with a Schottky field emission source. The accelerating voltage was 200 kV. The images obtained were selected from images of at least six different grid areas as representatives.

The length and diameter of the rod-shaped catalyst were measured using the scale in the software provided with the transmission scanning electron microscope image.

The analysis of the reaction product components was carried out on a gas chromatograph model 7890A purchased from Agilent.

The XRD diffraction spectrum was measured as follows: X-ray diffractometer (XRD) was manufactured by PANalytical, model Empyrean, Cu target generator, working tube voltage 40 KV, working tube current 40 mA, PixCel ^{3D} detector, divergence slit 1/4°, anti-scatter slit 1/2°, light bar 10 mm, Soller slit 0.04 rad, receiving slit 7.5 mm, scanning speed 0.013°/step, scanning time 30 s/step, scanning range: 5°-90°, and measurement was performed in reflection mode.

Sample preparation: selecting samples evenly, placing them in an agate mortar, grinding them thoroughly for 10 minutes, selecting 300-mesh powder after sieving, pressing the powder sample into the sample cell using the back pressure method, and measuring in reflection mode.

The lanthanum and doping element contents in the catalyst were measured by iCAP TQ ICP-MS purchased from Thermo Scientific.

The pore structure of the catalyst was characterized by BET analysis using an automatic adsorption instrument ASAP2420M purchased from MICROMERITICS, USA.

The methane conversion rate is calculated as follows: the methane conversion rate = amount of methane consumed in the reaction/initial amount of methane × 100%.

The ethylene selectivity is calculated as follows: the ethylene selectivity = amount of methane consumed for producing ethylene/total methane consumption × 100%.

The ethane selectivity is calculated as follows: the ethane selectivity = amount of methane consumed for producing ethane/total methane consumption × 100%.

The C2+ hydrocarbons selectivity includes the sum of ethylene, ethane, propylene, propane and higher carbon hydrocarbons.

The C2 hydrocarbon yield is calculated as follows: the C2 hydrocarbon yield = methane conversion rate × (ethane selectivity + ethylene selectivity).

### Example 1

3.15 g of lanthanum nitrate hexahydrate, 0.18 g of barium nitrate and 155 g of deionized water were accurately weighed and added into a beaker, and stirred at 900 rpm for 30 min at 25 °C to obtain a solution. Then, 10 wt% sodium hydroxide solution was added dropwise into the solution at 25 °C and 900 rpm. The first addition rate in terms of sodium hydroxide was 0.5 g/min per kilogram of the solution containing lanthanum nitrate and barium nitrate, that is, 0.5 g of sodium hydroxide was added per minute per kilogram of the solution containing lanthanum nitrate and barium nitrate. When the pH of the system was 9, the addition rate of the sodium hydroxide solution was increased to the second addition rate of 1 g/min in terms of sodium hydroxide per kilogram of the solution containing lanthanum nitrate and barium nitrate, that is, 1 g of sodium hydroxide was added per minute per kilogram of the solution containing lanthanum nitrate and barium nitrate. When the pH of the system was 12, the addition of the sodium hydroxide solution was stopped and the temperature was maintained at 25°C and the mixture was stirred for 10 min, and then the solution was heated to 100°C and condensed under stirring for reflux (dynamic aging, normal pressure) for 15 h. The stirring speed was 1500 rpm. After the solution was cooled to room temperature, the solid material was separated by centrifugation at 9000 rpm in a centrifuge and washed with deionized water until the pH value of the washing liquid was neutral. The obtained solid was dried at 80°C for 15 h and then calcined in air at 500°C for 2 h to prepare the lanthanum oxycarbonate catalyst.

According to ICP analysis, the content of lanthanum in the catalyst was 72.9 wt%, and the content of barium was 0.96 wt%, that is, the molar ratio of lanthanum to barium in the catalyst was 1:0.013.

BET analysis showed that the specific surface area of the lanthanum oxycarbonate catalyst was 46.36 m²/g, the pore volume was 0.23 cm³/g, and the average pore size was 201.48 nm.

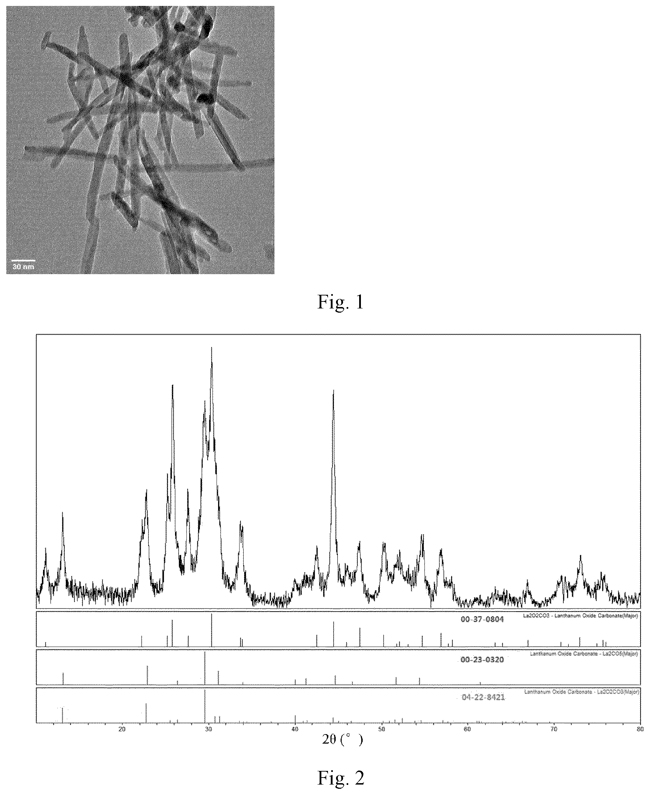

FIG 1 is a scanning electron microscope image of the lanthanum oxycarbonate catalyst prepared in Example 1. It can be seen from FIG 1 that the lanthanum oxycarbonate catalyst has a rod-like nanostructure, and it is calculated that the average diameter is 15 nm and the average aspect ratio is 20: 1.

FIG 2 is an XRD spectrum of the lanthanum oxycarbonate catalyst prepared in Example 1, wherein the abscissa is 2 θ and the ordinate is intensity. Compared with the PXRD database (Bruker Diffrac.Eva, version 4.2.1), the characteristic peaks attributed to the hexagonal phase lanthanum oxycarbonate in FIG 2 include 11.1° , 22.2° , 25.2° , 25.8° , 27.6° , 30.3° , 33.7° , 33.9° , 42.5° , 44.4° , 45.9° , 47.4° , 50.2° , 51.7° , 52.1° , 53.1° , 54.7° , 56.9° , 57.8° , 58.2° , 63.2° , 64.0° , 67.0° , 70. 8° , 71.6°, 73.0° , 75.0° , 75.7° , 76.0° ; the characteristic peaks attributed to tetragonal phase lanthanum oxycarbonate include 13.1 ° , 22.9°, 26.3° , 29.6° , 31.1° , 33.9° , 40.0° , 41.3° , 44.6° , 46.6° , 51.7° , 54.4° , 61.4° ; the characteristic peaks attributed to monoclinic phase lanthanum oxycarbonate include 13.1° , 22.8° , 25.5° , 26.4° , 29.5° , 30.7° , 31.3° , 33.5 ° , 34.0° , 34.4° , 37.2° , 40.0° , 40.9° , 41.4° , 44.4° , 45.0° , 46.4° , 50.2° , 50.9° , 51.6° , 52.4° , 53.9° , 54.6° , 57.1° , 57.7° , 61.0° , 61.3° , 61.6° , 64.0° , 64.4° , 65.3° , 66.3° , 66.7° , 69.5° ; there are no characteristic peaks attributed to elemental barium (characteristic peaks 25.1° , 35.8° , 44.2° ), barium oxide (characteristic peaks 26.7° , 34.7 ° , 40.4 ° , 41.0° ), barium hydroxide (characteristic peaks 26.3° , 269° , 27.4° , 34.2°), and barium carbonate (characteristic peaks at 22.1° , 25° , 36.5° , and 43.1 ° ). This indicates that the lanthanum oxycarbonate catalyst prepared in Example 1 contains hexagonal phase lanthanum oxycarbonate, tetragonal phase lanthanum oxycarbonate, and monoclinic phase lanthanum oxycarbonate.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.26; the molar ratio of the monoclinic phase lanthanum oxycarbonate to the tetragonal phase lanthanum oxycarbonate is 0.27:1.

### Example 2

3 g of lanthanum nitrate hexahydrate, 0.72 g of barium nitrate and 150 g of deionized water were accurately weighed and added into a beaker, and stirred at 900 rpm for 30 min at 30 °C to obtain a solution. Then, 15 wt% sodium hydroxide solution was added dropwise into the solution at 30 °C and 850 rpm. The first addition rate in terms of sodium hydroxide was 0.1 g/min per kilogram of the solution containing lanthanum nitrate and barium nitrate, that is, 0.1 g of sodium hydroxide was added per minute per kilogram of the solution containing lanthanum nitrate and barium nitrate. When the pH of the system was 8.5, the addition rate of the sodium hydroxide solution was increased to the second addition rate of 1 g/min in terms of sodium hydroxide per kilogram of the solution containing lanthanum nitrate and barium nitrate, that is, 1 g of sodium hydroxide was added per minute per kilogram of the solution containing lanthanum nitrate and barium nitrate. When the pH of the system was 11.8, the addition of the sodium hydroxide solution was stopped and the temperature was maintained at 30°C and the mixture was stirred for 10 min, and then the solution was heated to 90°C and condensed under stirring for reflux (dynamic aging, normal pressure) for 12 h. The stirring speed was 1200 rpm. After the solution was cooled to room temperature, the solid material was separated by centrifugation at 10000 rpm in a centrifuge and washed with deionized water until the pH value of the washing liquid was neutral. The solid was then washed once with ethanol. The obtained solid was dried at 100°C for 10 h and then calcined in air at 550°C for 4 h to prepare the lanthanum oxycarbonate catalyst.

According to ICP analysis, the content of lanthanum in the catalyst was 76.44 wt%, and the content of barium was 2.94 wt%, that is, the molar ratio of lanthanum to barium in the catalyst was 1:0.038.

BET analysis showed that the specific surface area of the lanthanum oxycarbonate catalyst was 48.3 m²/g, the pore volume was 0.26 cm³/g, and the average pore size was 200.1 nm.

It can be seen from the scanning electron microscope image (similar to Example 1, not shown) that the lanthanum oxycarbonate catalyst has a rod-like nanostructure, and it is calculated that the average diameter is 18 nm and the average aspect ratio is 26: 1.

The XRD spectrum of the lanthanum oxycarbonate catalyst prepared in Example 2 is similar to that of Example 1 (not shown), indicating that the lanthanum oxycarbonate catalyst prepared in Example 2 contains hexagonal phase lanthanum oxycarbonate, tetragonal phase lanthanum oxycarbonate and monoclinic phase lanthanum oxycarbonate.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.64; the molar ratio of the monoclinic phase lanthanum oxycarbonate to the tetragonal phase lanthanum oxycarbonate is 2.91: 1.

### Example 3

3 g of lanthanum nitrate hexahydrate, 0.54 g of barium nitrate and 150 g of deionized water were accurately weighed and added into a beaker, and stirred at 900 rpm for 30 min at 30 °C to obtain a solution. Then, 15 wt% sodium hydroxide solution was added dropwise into the solution at 35 °C and 950 rpm. The first addition rate in terms of sodium hydroxide was 0.6 g/min per kilogram of the solution containing lanthanum nitrate and barium nitrate, that is, 0.6 g of sodium hydroxide was added per minute per kilogram of the solution containing lanthanum nitrate and barium nitrate. When the pH of the system was 8, the addition rate of the sodium hydroxide solution was increased to the second addition rate of 5 g/min in terms of sodium hydroxide per kilogram of the solution containing lanthanum nitrate and barium nitrate, that is, 5 g of sodium hydroxide was added per minute per kilogram of the solution containing lanthanum nitrate and barium nitrate. When the pH of the system was 10.8, the addition of the sodium hydroxide solution was stopped and the temperature was maintained at 30°C and the mixture was stirred for 10 min, and then the solution was heated to 80°C and condensed under stirring for reflux (dynamic aging, normal pressure) for 12 h. The stirring speed was 1500 rpm. After the solution was cooled to room temperature, the solid material was separated by centrifugation at 10000 rpm in a centrifuge and washed with deionized water until the pH value of the washing liquid was neutral. The solid was then washed once with ethanol. The obtained solid was dried at 100°C for 10 h and then calcined in air at 480°C for 4 h to prepare the lanthanum oxycarbonate catalyst.

According to ICP analysis, the content of lanthanum in the catalyst was 75.48 wt%, and the content of barium was 2.04 wt%, that is, the molar ratio of lanthanum to barium in the catalyst was 1:0.027.

BET analysis showed that the specific surface area of the lanthanum oxycarbonate catalyst was 46.87 m²/g, the pore volume was 0.23 cm³/g, and the average pore size was 203 nm.

It can be seen from the scanning electron microscope image (similar to Example 1, not shown) that the lanthanum oxycarbonate catalyst has a rod-like nanostructure, and it is calculated that the average diameter is 21 nm and the average aspect ratio is 21: 1.

The XRD spectrum of the lanthanum oxycarbonate catalyst prepared in Example 3 is similar to that of Example 1 (not shown), indicating that the lanthanum oxycarbonate catalyst prepared in Example 3 contains hexagonal phase lanthanum oxycarbonate, tetragonal phase lanthanum oxycarbonate and monoclinic phase lanthanum oxycarbonate.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.49; the molar ratio of the monoclinic phase lanthanum oxycarbonate to the tetragonal phase lanthanum oxycarbonate is 1.34:1.

### Example 4

3 g of lanthanum nitrate hexahydrate, 0.18 g of magnesium nitrate hexahydrate and 150 g of deionized water were accurately weighed and added into a beaker, and stirred at 900 rpm for 30 min at 30 °C to obtain a solution. Then, 15 wt% sodium hydroxide solution was added dropwise into the solution at 30 °C and 950 rpm. The first addition rate in terms of sodium hydroxide was 0.6 g/min per kilogram of the solution containing lanthanum nitrate and magnesium nitrate, that is, 0.6 g of sodium hydroxide was added per minute per kilogram of the solution containing lanthanum nitrate and magnesium nitrate. When the pH of the system was 8.8, the addition rate of the sodium hydroxide solution was increased to the second addition rate of 2 g/min in terms of sodium hydroxide per kilogram of the solution containing lanthanum nitrate and magnesium nitrate, that is, 2 g of sodium hydroxide was added per minute per kilogram of the solution containing lanthanum nitrate and magnesium nitrate. When the pH of the system was 11.6, the addition of the sodium hydroxide solution was stopped and the temperature was maintained at 30°C and the mixture was stirred for 10 min, and then the solution was heated to 90°C and condensed under stirring for reflux (dynamic aging, normal pressure) for 12 h. The stirring speed was 1250 rpm. After the solution was cooled to room temperature, the solid material was separated by centrifugation at 10000 rpm in a centrifuge and washed with deionized water until the pH value of the washing liquid was neutral. The solid was then washed once with ethanol. The obtained solid was dried at 100°C for 10 h and then calcined in air at 475°C for 4 h to prepare the lanthanum oxycarbonate catalyst.

According to ICP analysis, the content of lanthanum in the catalyst was 72.3 wt%, and the content of magnesium was 0.75 wt%, that is, the molar ratio of lanthanum to magnesium in the catalyst was 1:0.059.

BET analysis showed that the specific surface area of the lanthanum oxycarbonate catalyst was 72.93 m²/g, the pore volume was 0.42 cm³/g, and the average pore size was 184.2 nm.

It can be seen from the scanning electron microscope image (similar to Example 1, not shown) that the lanthanum oxycarbonate catalyst has a rod-like nanostructure, and it is calculated that the average diameter is 25 nm and the average aspect ratio is 20: 1.

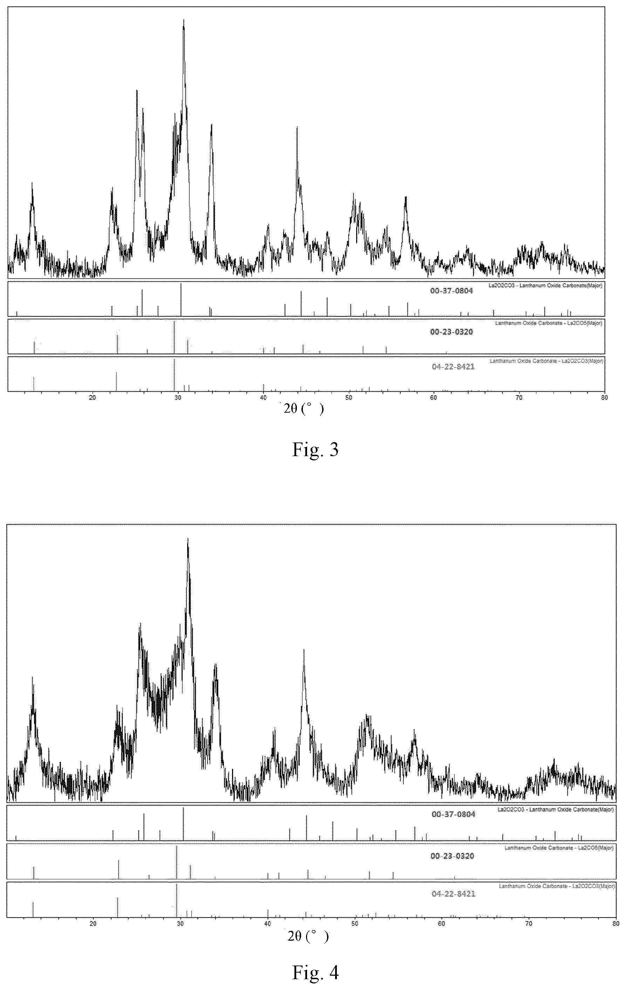

FIG 3 is an XRD spectrum of the lanthanum oxycarbonate catalyst prepared in Example 4, wherein the abscissa is 2 θ and the ordinate is intensity. Compared with the PXRD database (Bruker Diffrac.Eva, version 4.2.1), FIG 3 has the characteristic peaks attributed to the hexagonal phase lanthanum oxycarbonate (the same as the characteristic peaks attributed to the hexagonal phase lanthanum oxycarbonate in Example 1), the characteristic peaks attributed to the tetragonal phase lanthanum oxycarbonate (the same as the characteristic peaks attributed to the tetragonal phase lanthanum oxycarbonate in Example 1), and the characteristic peaks attributed to the monoclinic phase lanthanum oxycarbonate (the same as the characteristic peaks attributed to the monoclinic phase lanthanum oxycarbonate in Example 1); there are no characteristic peaks attributed to elemental magnesium (characteristic peaks 32.2° , 34.4 ° , 36.6° , 63.1° ), magnesium oxide (characteristic peaks 36.9° , 42.8° , 62.2° , 78.4° ), magnesium hydroxide (characteristic peaks 18.6° , 38.0° , 50.9° , 58.7° ), and magnesium carbonate (characteristic peaks 32.6° , 43.0° , 539° , 70.3° ). This indicates that the lanthanum oxycarbonate catalyst prepared in Example 4 contains hexagonal phase lanthanum oxycarbonate, tetragonal phase lanthanum oxycarbonate, and monoclinic phase lanthanum oxycarbonate.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.67; the molar ratio of the monoclinic phase lanthanum oxycarbonate to the tetragonal phase lanthanum oxycarbonate is 0.70:1.

### Example 5 - adding sodium hydroxide solution at a constant rate

3.15 g of lanthanum nitrate hexahydrate, 0.18 g of barium nitrate and 155 g of deionized water were accurately weighed and added into a beaker, and stirred at 900 rpm for 30 min at 25 °C to obtain a solution. Then, 10 wt% sodium hydroxide solution was added dropwise into the solution at 25 °C and 900 rpm. The addition rate in terms of sodium hydroxide was 2 g/min per kilogram of the solution containing lanthanum nitrate and barium nitrate, that is, 2 g of sodium hydroxide was added per minute per kilogram of the solution containing lanthanum nitrate and barium nitrate. When the pH of the system was 12, the addition of the sodium hydroxide solution was stopped and the temperature was maintained at 25°C and the mixture was stirred for 10 min, and then the solution was heated to 100°C and condensed under stirring for reflux (dynamic aging, normal pressure) for 15 h. The stirring speed was 1500 rpm. After the solution was cooled to room temperature, the solid material was separated by centrifugation at 9000 rpm in a centrifuge and washed with deionized water until the pH value of the washing liquid was neutral. The obtained solid was dried at 80°C for 15 h and then calcined in air at 500°C for 2 h to prepare the lanthanum oxycarbonate catalyst.

According to ICP analysis, the content of lanthanum in the catalyst was 76.44 wt%, and the content of barium was 0.9 wt%, that is, the molar ratio of lanthanum to barium in the catalyst was 1:0.012.

BET analysis showed that the specific surface area of the lanthanum oxycarbonate catalyst was 74.1 m²/g, the pore volume was 0.46 cm³/g, and the average pore size was 111.6 nm.

It can be seen from the scanning electron microscope image (similar to Example 1, not shown) that the lanthanum oxycarbonate catalyst has a rod-like nanostructure, and it is calculated that the average diameter is 24 nm and the average aspect ratio is 16.7:1.

The XRD spectrum of the lanthanum oxycarbonate catalyst prepared in Example 5 is similar to that of Example 1 (not shown), indicating that the lanthanum oxycarbonate catalyst prepared in Example 5 contains hexagonal phase lanthanum oxycarbonate, tetragonal phase lanthanum oxycarbonate and monoclinic phase lanthanum oxycarbonate.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.2; the molar ratio of the monoclinic phase lanthanum oxycarbonate to the tetragonal phase lanthanum oxycarbonate is 1.75:1.

### Example 6 - hydrothermal method

3.15 g of lanthanum nitrate hexahydrate, 0.18 g of barium nitrate and 155 g of deionized water were accurately weighed and added into a beaker, and stirred at 900 rpm for 30 min at 25 °C to obtain a solution. Then, 10 wt% sodium hydroxide solution was added dropwise into the solution at 25 °C and 920 rpm. The first addition rate in terms of sodium hydroxide was 1 g/min per kilogram of the solution containing lanthanum nitrate and barium nitrate, that is, 1 g of sodium hydroxide was added per minute per kilogram of the solution containing lanthanum nitrate and barium nitrate. When the pH of the system was 8.6, the addition rate of the sodium hydroxide solution was increased to the second addition rate of 5 g/min in terms of sodium hydroxide per kilogram of the solution containing lanthanum nitrate and barium nitrate, that is, 5 g of sodium hydroxide was added per minute per kilogram of the solution containing lanthanum nitrate and barium nitrate. When the pH of the system was 12, the addition of the sodium hydroxide solution was stopped and the temperature was maintained at 25°C and the mixture was stirred for 10 min. Then the solution was transferred to a hydrothermal autoclave lined with polytetrafluoroethylene and statically reacted at 180°C for 15 hours. After the hydrothermal autoclave was cooled to room temperature, the solid material was separated by centrifugation at 9000 rpm in a centrifuge and washed with deionized water until the pH value of the washing liquid was neutral. The obtained solid was dried at 80°C for 15 h and then calcined in air at 500°C for 2 h to prepare the lanthanum oxycarbonate catalyst.

According to ICP analysis, the content of lanthanum in the catalyst was 72 wt%, and the content of barium was 1.2 wt%, that is, the molar ratio of lanthanum to barium in the catalyst was 1:0.017.

BET analysis showed that the specific surface area of the lanthanum oxycarbonate catalyst was 64.2 m²/g, the pore volume was 0.5 cm³/g, and the average pore size was 253 nm.

It can be seen from the scanning electron microscope image (similar to Example 1, not shown) that the lanthanum oxycarbonate catalyst has a rod-like nanostructure, and it is calculated that the average diameter is 26 nm and the average aspect ratio is 15: 1.

FIG 4 is an XRD spectrum of the lanthanum oxycarbonate catalyst prepared in Example 6, wherein the abscissa is 2 θ and the ordinate is intensity. Compared with the PXRD database (Bruker Diffrac.Eva, version 4.2.1), FIG 4 has the characteristic peaks attributed to the hexagonal phase lanthanum oxycarbonate (the same as the characteristic peaks attributed to the hexagonal phase lanthanum oxycarbonate in Example 1), the characteristic peaks attributed to the tetragonal phase lanthanum oxycarbonate (the same as the characteristic peaks attributed to the tetragonal phase lanthanum oxycarbonate in Example 1), and the characteristic peaks attributed to the monoclinic phase lanthanum oxycarbonate (the same as the characteristic peaks attributed to the monoclinic phase lanthanum oxycarbonate in Example 1); there are no characteristic peaks attributed to elemental barium (characteristic peaks 25.1° , 35.8° , 44.2° ), barium oxide (characteristic peaks 26.7° , 34.7° , 40.4° , 41.0° ), barium hydroxide (characteristic peaks 26.3° , 26.9° , 27.4° , 34.2° ), and barium carbonate (characteristic peaks 22.1° , 25° , 36.5° , 43.1° ). This indicates that the lanthanum oxycarbonate catalyst prepared in Example 6 contains hexagonal phase lanthanum oxycarbonate, tetragonal phase lanthanum oxycarbonate, and monoclinic phase lanthanum oxycarbonate.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.3; the molar ratio of the monoclinic phase lanthanum oxycarbonate to the tetragonal phase lanthanum oxycarbonate is 0.41:1.

### Example 7

3.15 g of lanthanum nitrate hexahydrate, 0.18 g of strontium nitrate and 155 g of deionized water were accurately weighed and added into a beaker, and stirred at 900 rpm for 30 min at 25 °C to obtain a solution. Then, 10 wt% sodium hydroxide solution was added dropwise into the solution at 25 °C and 900 rpm. The first addition rate in terms of sodium hydroxide was 0.1 g/min per kilogram of the solution containing lanthanum nitrate and strontium nitrate, that is, 0.1 g of sodium hydroxide was added per minute per kilogram of the solution containing lanthanum nitrate and strontium nitrate. When the pH of the system was 9, the addition rate of the sodium hydroxide solution was increased to the second addition rate of 2 g/min in terms of sodium hydroxide per kilogram of the solution containing lanthanum nitrate and strontium nitrate, that is, 2 g of sodium hydroxide was added per minute per kilogram of the solution containing lanthanum nitrate and strontium nitrate. When the pH of the system was 12, the addition of the sodium hydroxide solution was stopped and the temperature was maintained at 25°C and the mixture was stirred for 10 min, and then the solution was heated to 80°C and condensed under stirring for reflux (dynamic aging, normal pressure) for 15 h. The stirring speed was 1500 rpm. After the solution was cooled to room temperature, the solid material was separated by centrifugation at 9000 rpm in a centrifuge and washed with deionized water until the pH value of the washing liquid was neutral. The obtained solid was dried at 80°C for 15 h and then calcined in air at 500°C for 2 h to prepare the lanthanum oxycarbonate catalyst doped with strontium element.

According to ICP analysis, the content of lanthanum in the catalyst was 71.3 wt%, and the content of strontium was 0.99 wt%, that is, the molar ratio of lanthanum to strontium in the catalyst was 1:0.022.

BET analysis showed that the specific surface area of the lanthanum oxycarbonate catalyst was 57 m²/g, the pore volume was 0.37 cm³/g, and the average pore size was 253 nm.

It can be seen from the scanning electron microscope image (similar to Example 1, not shown) that the lanthanum oxycarbonate catalyst has a rod-like nanostructure, and it is calculated that the average diameter is 30 nm and the average aspect ratio is 14: 1.

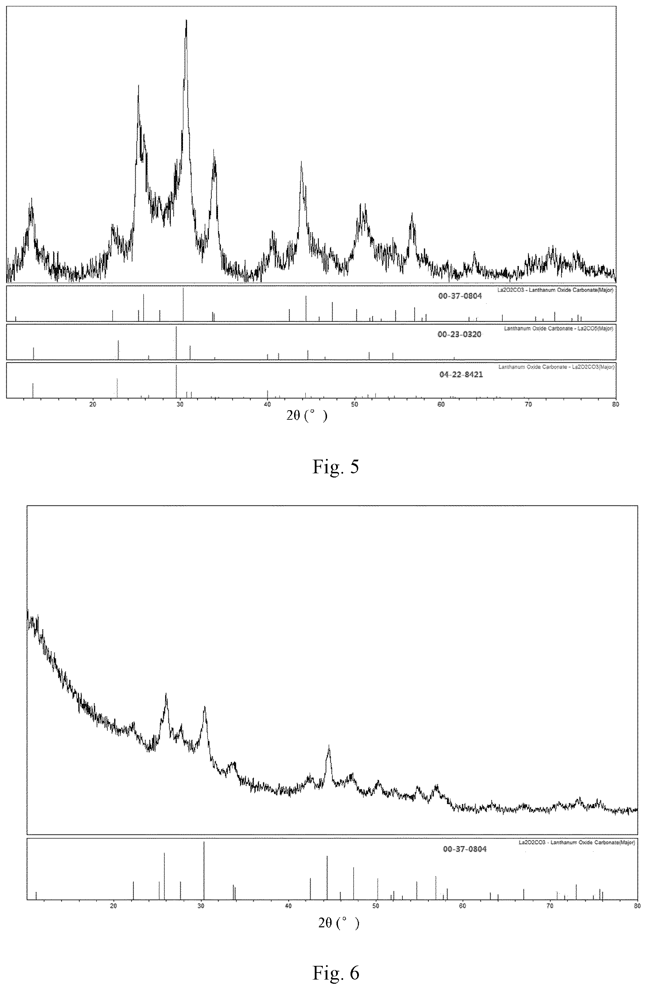

FIG 5 is an XRD spectrum of the lanthanum oxycarbonate catalyst prepared in Example 7, wherein the abscissa is 2 θ and the ordinate is intensity. Compared with the PXRD database (Bruker Diffrac.Eva, version 4.2.1), FIG 5 has the characteristic peaks attributed to the hexagonal phase lanthanum oxycarbonate (the same as the characteristic peaks attributed to the hexagonal phase lanthanum oxycarbonate in Example 1), the characteristic peaks attributed to the tetragonal phase lanthanum oxycarbonate (the same as the characteristic peaks attributed to the tetragonal phase lanthanum oxycarbonate in Example 1), and the characteristic peaks attributed to the monoclinic phase lanthanum oxycarbonate (the same as the characteristic peaks attributed to the monoclinic phase lanthanum oxycarbonate in Example 1); there are no characteristic peaks attributed to elemental strontium (characteristic peaks 25.4° , 29.4° , 42.0° , 49.7° ), strontium oxide (characteristic peaks 27.2 ° , 28.6° , 35.7° , 45.5° ), strontium hydroxide hydrate (characteristic peaks 19.5 ° , 31.8 ° , 39.2 ° , 40.4 ° ), and strontium carbonate (characteristic peaks 22.2° , 27.5° , 43.1° , 45.6° ). This indicates that the lanthanum oxycarbonate catalyst prepared in Example 7 contains hexagonal phase lanthanum oxycarbonate, tetragonal phase lanthanum oxycarbonate, and monoclinic phase lanthanum oxycarbonate.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.9; the molar ratio of the monoclinic phase lanthanum oxycarbonate to the tetragonal phase lanthanum oxycarbonate is 1.37:1.

### Example 8

The method of Example 1 was followed, except that "0.18 g of barium nitrate" was replaced by "0.23 g of cerium nitrate".

According to ICP analysis, the content of lanthanum in the catalyst was 69.2 wt%, and the content of cerium was 3.2 wt%, that is, the molar ratio of lanthanum to cerium in the catalyst was 1:0.045.

BET analysis showed that the specific surface area of the lanthanum oxycarbonate catalyst was 79.2 m²/g, the pore volume was 0.43 cm³/g, and the average pore size was 211 nm.

It can be seen from the scanning electron microscope image (similar to Example 1, not shown) that the lanthanum oxycarbonate catalyst has a rod-like nanostructure, and it is calculated that the average diameter is 19 nm and the average aspect ratio is 25: 1.

The XRD spectrum of the lanthanum oxycarbonate catalyst prepared in Example 8 is similar to that of Example 1 (not shown). It is known from the spectrum that the lanthanum oxycarbonate catalyst prepared in Example 8 has the characteristic peaks attributed to the hexagonal phase lanthanum oxycarbonate (the same as the characteristic peaks attributed to the hexagonal phase lanthanum oxycarbonate in Example 1), the characteristic peaks attributed to the tetragonal phase lanthanum oxycarbonate (the same as the characteristic peaks attributed to the tetragonal phase lanthanum oxycarbonate in Example 1), and the characteristic peaks attributed to the monoclinic phase lanthanum oxycarbonate (the same as the characteristic peaks attributed to the monoclinic phase lanthanum oxycarbonate in Example 1); there are no characteristic peaks attributed to elemental cerium (characteristic peaks 30.0° , 34.8° , 50.1° , 59.5° ), cerium oxide (characteristic peaks 28.5° , 33.1° , 47.5° , 56.3° ), cerium hydroxide (characteristic peaks 15.8° , 27.4° , 28.2° , 48.9° ), and cerium carbonate (characteristic peaks 17.0° , 29.7° , 34.5° , 42.6° ). This indicates that the lanthanum oxycarbonate catalyst prepared in Example 8 contains hexagonal phase lanthanum oxycarbonate, tetragonal phase lanthanum oxycarbonate, and monoclinic phase lanthanum oxycarbonate.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.7; the molar ratio of the monoclinic phase lanthanum oxycarbonate to the tetragonal phase lanthanum oxycarbonate is 1.10:1.

### Comparative Example 1

3.15 g of lanthanum nitrate hexahydrate and 155 g of deionized water were accurately weighed and added into a beaker, and stirred at 900 rpm for 30 min at 25 °C to obtain a solution. Then, 10 wt% sodium hydroxide solution was added dropwise into the solution at 25 °C and 900 rpm. The addition rate in terms of sodium hydroxide was 0.5 g/min per kilogram of the solution containing lanthanum nitrate, that is, 0.5 g of sodium hydroxide was added per minute per kilogram of the solution containing lanthanum nitrate. When the pH of the system was 12, the addition of the sodium hydroxide solution was stopped and the temperature was maintained at 25°C and the mixture was stirred for 10 min, and then the solution was heated to 100°C and condensed under stirring for reflux (dynamic aging) for 15 h. The stirring speed was 1200 rpm. After the solution was cooled to room temperature, the solid material was separated by centrifugation at 9000 rpm in a centrifuge and washed with deionized water until the pH value of the washing liquid was neutral. The obtained solid was dried at 80°C for 15 h and then calcined in air at 500°C for 2 h to prepare the lanthanum oxycarbonate catalyst.

According to ICP analysis, the content of lanthanum in the catalyst was 85 wt%.

BET analysis showed that the specific surface area of the lanthanum oxycarbonate catalyst was 83 m²/g, the pore volume was 0.43 cm³/g, and the average pore size was 95 nm.

It can be seen from the scanning electron microscope image (similar to Example 1, not shown) that the lanthanum oxycarbonate catalyst has a rod-like nanostructure, and it is calculated that the average diameter is 20 nm and the average aspect ratio is 20: 1.

FIG 6 is an XRD spectrum of the lanthanum oxycarbonate catalyst prepared in Comparative Example 1, wherein the abscissa is 2 θ and the ordinate is intensity. Compared with the PXRD database (Bruker Diffrac.Eva, version 4.2.1), FIG 6 only has the characteristic peaks attributed to the hexagonal phase lanthanum oxycarbonate (the same as the characteristic peaks attributed to the hexagonal phase lanthanum oxycarbonate in Example 1). This indicates that the lanthanum oxycarbonate catalyst prepared in Comparative Example 1 is pure hexagonal phase lanthanum oxycarbonate.

### Example 9

The method of Example 1 was followed, except that the aging temperature was 40°C.

According to ICP analysis, the content of lanthanum in the catalyst was 73 wt%, and the content of barium was 2.1 wt%, that is, the molar ratio of lanthanum to barium in the catalyst was 1:0.03.

BET analysis showed that the specific surface area of the lanthanum oxycarbonate catalyst was 45 m²/g, the pore volume was 0.3 cm³/g, and the average pore size was 86 nm.

It can be seen from the scanning electron microscope image (similar to Example 1, not shown) that the lanthanum oxycarbonate catalyst has a rod-like nanostructure, and it is calculated that the average diameter is 16 nm and the average aspect ratio is 20:1; the molar ratio of the monoclinic phase lanthanum oxycarbonate to the tetragonal phase lanthanum oxycarbonate is 0.79:1.

### Example 10

The method of Example 1 was followed, except that the first addition rate was 5 g/min and the second addition rate was 0.5 g/min.

According to ICP analysis, the content of lanthanum in the catalyst was 66 wt%, and the content of barium was 3 wt%, that is, the molar ratio of lanthanum to barium in the catalyst was 1:0.045.

BET analysis showed that the specific surface area of the lanthanum oxycarbonate catalyst was 49 m²/g, the pore volume was 0.2 cm³/g, and the average pore size was 45 nm.

It can be seen from the scanning electron microscope image (similar to Example 1, not shown) that the lanthanum oxycarbonate catalyst has a rod-like nanostructure, and it is calculated that the average diameter is 13 nm and the average aspect ratio is 25: 1.

The XRD spectrum of the lanthanum oxycarbonate catalyst prepared in Example 10 is similar to that of Example 1 and is not shown again.

According to the XRD full spectrum fitting method, it is calculated that the ratio of the molar amount of the hexagonal phase lanthanum oxycarbonate to the total molar amount of the tetragonal phase lanthanum oxycarbonate and the monoclinic phase lanthanum oxycarbonate is 1:0.2; the molar ratio of the monoclinic phase lanthanum oxycarbonate to the tetragonal phase lanthanum oxycarbonate is 0.57:1.

### Comparative Example 2

The method of Example 1 was followed, except that 10 wt% sodium hydroxide solution was added dropwise to the solution at 25°C and 900 rpm, and the addition rate in terms of sodium hydroxide was 0.5 g/min per kilogram of the solution containing lanthanum nitrate and barium nitrate, that is, 0.5 g of sodium hydroxide was added per minute per kilogram of the solution containing lanthanum nitrate and barium nitrate. When the pH of the system was 8, the addition of the sodium hydroxide solution was stopped.

The catalyst prepared by the method of Comparative Example 2 does not have a rod-like structure, and no hexagonal phase lanthanum oxycarbonate, tetragonal phase lanthanum oxycarbonate and monoclinic phase lanthanum oxycarbonate are found.

### Comparative Example 3

1.95 g of lanthanum nitrate hexahydrate and 5.4 g of urea were weighted, dissolved in 300 ml of deionized water, and stirred until completely dissolved. Aqueous ammonia was added to the resulting solution to adjust the pH value to 8.5. The mixture was stirred for 3 h in a 90°C oil bath. The resulting white suspension was cooled naturally to room temperature and centrifuged. The resulting precipitate was washed twice with anhydrous ethanol and centrifuged. The white precipitate was dried at 80°C for 12 h, and then calcined at 500°C for 2 h to prepare the lanthanum oxycarbonate.

0.018 g of cerium nitrate hexahydrate was completely dissolved in deionized water, and 0.105 g of the lanthanum oxycarbonate was impregnated with the obtained solution, wherein the molar ratio of cerium nitrate hexahydrate to the lanthanum oxycarbonate was 0.15:1. The obtained sample was completely dried at 80°C and calcined at 600°C for 2h to prepare a solid sample.

XRD analysis showed that the obtained solid had hexagonal phase lanthanum oxycarbonate and monoclinic phase lanthanum oxycarbonate, but did not have tetragonal phase lanthanum oxycarbonate.

### Test Example 1

The catalysts directly obtained from the preparation process of the above examples and comparative examples were in the form of particles, which were pressed into tablets and sieved through 40-60 mesh. Then 0.1 g was taken and loaded into an Inconel fixed bed reactor. Under normal pressure, methane and oxygen were introduced to react. Other reaction conditions and results are shown in Table 1.

**Table 1**

| Catalyst | Reaction temperature °C | Alkane oxygen ratio / | Methane space velocity ml/gh | Methane conversion rate % | Selectivity of C2 hydrocarbon % | Yield of C2 hydrocarbon % |
|---|---|---|---|---|---|---|
| Example 1 | 450 | 3:1 | 40000 | 25.4 | 55.7 | 14.1 |
| | 500 | 3:1 | 40000 | 26.9 | 57.6 | 15.5 |
| | 600 | 3:1 | 40000 | 31.3 | 51.1 | 16.0 |
| | 650 | 3:1 | 40000 | 32.5 | 46.3 | 15.0 |
| Example 2 | 500 | 3:1 | 40000 | 27.1 | 56.5 | 15.3 |
| Example 3 | 600 | 3:1 | 40000 | 32.4 | 50.1 | 16.2 |
| Example 4 | 600 | 3:1 | 40000 | 30.7 | 52.6 | 16.1 |
| Example 5 | 600 | 3:1 | 40000 | 25 | 53.8 | 13.5 |
| Example 6 | 450 | 3:1 | 40000 | 25.3 | 58.9 | 14.9 |
| | 500 | 3:1 | 40000 | 26.4 | 57.3 | 15.1 |
| | 600 | 3:1 | 40000 | 27.8 | 56.2 | 15.6 |
| Example 7 | 450 | 3:1 | 40000 | 25.7 | 57.9 | 14.9 |
| Example 8 | 500 | 3:1 | 40000 | 26.4 | 56.7 | 15.0 |
| Comparative Example 1 | 450 | 3:1 | 40000 | 24.7 | 47.8 | 11.8 |
| Example 9 | 500 | 3:1 | 40000 | 28.1 | 36.7 | 10.2 |
| Example 10 | 500 | 3:1 | 40000 | 23.4 | 48.7 | 11.4 |
| Comparative Example 2 | 500 | 3:1 | 40000 | 12.3 | 36.2 | 4.45 |

### Test Example 2

0.1 g of the powder in the form of particles directly obtained from the preparation process of Example 1 was directly taken and loaded into an Inconel fixed bed reactor. The upper and lower sides of the catalyst were fixed in position using quartz wool. Under normal pressure, methane and oxygen were introduced to react. The reaction conditions and results are shown in Table 2.

**Table 2**

| Catalyst | Reaction temperature | Alkane oxygen ratio | Methane space velocity | Methane conversion rate | Selectivity of C2 hydrocarbon | Yield of C2 hydrocarbon |
|---|---|---|---|---|---|---|
| | °C | / | ml /gh | % | % | % |
| Example 1 | 450 | 3:1 | 40000 | 25.5 | 55.4 | 14.13 |
| | 500 | 3:1 | 40000 | 26.8 | 57.7 | 15.46 |
| | 600 | 3:1 | 40000 | 31.5 | 50.9 | 16.0 |
| | 650 | 3:1 | 40000 | 32.3 | 46.4 | 14.99 |

The short-term catalytic performance of the powder and the tablet is not much different. The difference is that the powder has a small particle size, which leads to a high bed pressure, which is a problem in engineering scale-up.

It can be seen from the results in Table 1 that, compared with Comparative Examples 1-2, the catalyst containing hexagonal phase lanthanum oxycarbonate, tetragonal phase lanthanum oxycarbonate and monoclinic phase lanthanum oxycarbonate prepared by the present invention can have a higher C2 hydrocarbon yield at low temperature when used in the methane oxidative coupling reaction. Furthermore, by using the preferred embodiments of the present invention, Examples 1-4 and Examples 6-8 can obtain a higher C2 hydrocarbon yield.

The preferred embodiments of the present invention are described in detail above, but the present invention is not limited thereto. Within the technical concept of the present invention, the technical solution of the present invention can be subjected to a variety of simple modifications, including the combination of various technical features in any other suitable manner, and these simple modifications and combinations should also be regarded as the contents disclosed by the present invention and belong to the protection scope of the present invention.

## Claims

1. A lanthanum oxycarbonate catalyst, **characterized in that** the lanthanum oxycarbonate catalyst comprises the following three crystal phases coexisting in a crystalline state: hexagonal phase lanthanum oxycarbonate, monoclinic phase lanthanum oxycarbonate containing a doping element, and tetragonal phase lanthanum oxycarbonate containing a doping element, wherein the ratio of the molar amount of the hexagonal phase lanthanum oxycarbonate to the total molar amount of the tetragonal phase lanthanum oxycarbonate and the monoclinic phase lanthanum oxycarbonate is 1:0.01-1 and the molar ratio of the tetragonal phase lanthanum oxycarbonate and the monoclinic phase lanthanum oxycarbonate is 1:0.1-10.

2. The lanthanum oxycarbonate catalyst according to claim 1, wherein the doping element in the monoclinic phase lanthanum oxycarbonate and the tetragonal phase lanthanum oxycarbonate is at least one selected from the group consisting of elements of Groups IIA, VIII, IB, IIB and lanthanide elements other than lanthanum, preferably at least one of Mg, Ca, Sr, Ba, Fe, Zn, Ce and Cd, more preferably at least one of Mg, Ca, Sr and Ba;
and/or, the molar ratio of lanthanum element to the doping element in the lanthanum oxycarbonate catalyst is 1:0.01-0.1, preferably 1:0.01-0.08, and more preferably 1:0.012-0.06.

3. The lanthanum oxycarbonate catalyst according to claim 1, wherein the ratio of the molar amount of the hexagonal phase lanthanum oxycarbonate to the total molar amount of the tetragonal phase lanthanum oxycarbonate and the monoclinic phase lanthanum oxycarbonate is 1:0.1-1, more preferably 1:0.25-0.9; and/or
the molar ratio of the tetragonal phase lanthanum oxycarbonate and the monoclinic phase lanthanum oxycarbonate is 1:0.2-5, preferably 1:0.25-3.

4. The lanthanum oxycarbonate catalyst according to any one of claims 1 to 3, wherein the lanthanum oxycarbonate catalyst has a nanostructure, preferably the catalyst is in a rod form with a hydraulic diameter of 10 nm-40 nm, preferably 10 nm-30 nm, preferably 15 nm-30 nm; or preferably the catalyst is in a flake form with a thickness of 100 nm-500 nm, preferably 200 nm-400 nm; or preferably the catalyst is in a spherical form with a hydraulic diameter of 100 nm-800 nm, preferably 200 nm-600 nm.

5. The lanthanum oxycarbonate catalyst according to any one of claims 1 to 3, wherein the lanthanum oxycarbonate catalyst has a specific surface area of 40 m²/g-100 m²/g, a pore volume of 0.2 cm³/g-0.7 cm³/g, and an average pore size of 5 nm-300 nm, preferably 8 nm-290 nm, and more preferably 180 nm-260 nm.

6. The lanthanum oxycarbonate catalyst according to any one of claims 1 to 3, wherein the lanthanum oxycarbonate catalyst has a rod-shaped nanostructure, a diameter of 10 nm-30 nm, and an aspect ratio of 5-30: 1, preferably 10-26: 1.

7. A method for preparing the lanthanum oxycarbonate catalyst as claimed in any one of claims 1 to 6, **characterized in that** the method comprises: adding an alkali solution to a mixed solution containing a lanthanum source and a compound containing a doping element to make the pH value of the mixed system greater than 9, and then sequentially performing reaction, solid-liquid separation, drying and calcination;
wherein, relative to each kilogram of the mixed solution, the addition rate of the alkali solution is 0.01 g/min-10 g/min, preferably 0.03 g/min-8 g/min, and more preferably 0.5 g/min-6 g/min, based on the alkali in the alkali solution; and wherein, firstly, the alkali solution is added dropwise to the mixed solution at a first addition rate until the pH value of the solution reaches a set value, which is between 8 and 9, and then the alkali solution is added dropwise to the mixed solution at a second addition rate until the pH value of the system is 10-13, wherein the second addition rate is 0.5-4.5 g/min higher than the first addition rate.

8. The method according to claim 7, wherein the lanthanum source is a water-soluble salt of lanthanum, preferably at least one of lanthanum nitrate, lanthanum chloride and lanthanum acetate, and more preferably lanthanum nitrate.

9. The method according to claim 7 or 8, wherein the concentration of the alkali solution is 2 wt%-25 wt%, preferably 3 wt%-20 wt%, and more preferably 5 wt%-18 wt%.

10. The method according to claim 7 or 8, wherein the alkali in the alkali solution is a compound of a Group IA metal.

11. The method according to claim 7, wherein the first addition rate is 0.01-1 g/min; and/or the second addition rate is 0.5-5 g/min.

12. The method according to claim 7 or 8, wherein the reaction temperature is 80-200°C and the reaction time is 10h-50h;
and/or, the drying temperature is 60°C-100°C, and the drying time is 12h -24h;
and/or, the calcination temperature is 450°C-550°C, and the calcination time is 2h-8h.

13. The method according to claim 7 or 8, wherein the method further comprises:
S1. under a first stirring condition, mixing the lanthanum source and the compound containing the doping element with water to obtain a mixed solution; preferably, the first stirring condition includes: a temperature of 10-50 °C, a rotation speed of 300-1000rpm, and a time of 10-40min;
S2. under a second stirring condition, adding the alkali solution to the mixed solution to obtain a mixed system; preferably, the second stirring condition includes: a temperature of 20-60 °C and a rotation speed of 500-1500 rpm.

14. A lanthanum oxycarbonate catalyst composition, comprising the lanthanum oxycarbonate catalyst according to any one of claims 1 to 6 and a binder, wherein the binder comprises alumina, soluble starch, silica sol and polyethylene glycol.

15. A lanthanum oxycarbonate catalyst composition, comprising the lanthanum oxycarbonate catalyst according to any one of claims 1 to 6 and a support, wherein the support comprises alumina, silica, molecular sieve, diatomaceous earth and ceramic.

16. Use of the lanthanum oxycarbonate catalyst according to any one of claims 1 to 6, the lanthanum oxycarbonate catalyst composition according to claim 14, or the lanthanum oxycarbonate catalyst composition according to claim 15 in the oxidative coupling reaction of methane to produce C2+ hydrocarbons.

17. A method for preparing C2+ hydrocarbons from methane, **characterized in that** the method comprises: contacting methane with the lanthanum oxycarbonate catalyst according to any one of claims 1 to 6, the lanthanum oxycarbonate catalyst composition according to claim 14, or the lanthanum oxycarbonate catalyst composition according to claim 15 for reaction in the presence of oxygen and under the conditions of methane oxidative coupling reaction.

18. The method according to claim 17, wherein the molar ratio of methane to oxygen is 2-9: 1;
and/or, the contact and reaction temperature is 450°C-650°C;
and/or, the space velocity of methane is 5000 mL/(g·h)-200000 mL/(g ·h).
